# EUROPEAN PATENT SPECIFICATION

(11) **EP 4 190 251 B1**
(45) Date of publication and mention of the grant of the patent: **02.04.2025**
(21) Application number: 21854130.8
(22) Date of filing: 06.08.2021
(51) Int. Cl.: A61B 17/326, A61B 17/115, A61B 17/064

(54) **CIRCUMCISION ANASTOMAT**
BESCHNEIDUNGSANASTOMAT
DISPOSITIF D'ANASTOMOSE DE CIRCONCISION

(30) Priority: 07.08.2020 CN 202021629407 U
(43) Date of publication of application: 07.06.2023
(73) Proprietor: Zhejiang Yigao Medical Technology Co., Ltd., Hangzhou, Zhejiang 311200 (CN)
(72) Inventor: LI, Fangbing, Hangzhou, Zhejiang 311200 (CN); ZHENG, Peng, Hangzhou, Zhejiang 311200 (CN); ZHENG, Xingrong, Hangzhou, Zhejiang 311200 (CN); PENG, Shouqiang, Hangzhou, Zhejiang 311200 (CN); ZHANG, Ziyang, Hangzhou, Zhejiang 311200 (CN); LI, Xueying, Hangzhou, Zhejiang 311200 (CN); ZHOU, Lingqiang, Hangzhou, Zhejiang 311200 (CN); WU, Qiuju, Hangzhou, Zhejiang 311200 (CN)
(74) Representative: Sun, Yiming
(86) International application number: PCT/CN2021/111138
(87) International publication number: WO 2022/028567

(56) References cited:
- EP-A1- 3 607 890
- WO-A1-2015/010477
- CN-A- 101 534 724
- CN-A- 105 232 100
- CN-A- 111 759 412
- CN-U- 205 181 412
- CN-U- 205 964 118
- CN-U- 211 066 909
- CN-U- 213 606 718
- CN-U- 213 606 719
- CN-U- 213 606 720
- US-A1- 2008 065 154
- US-A1- 2014 263 570

## Description

### TECHNICAL FIELD

The present invention relates to circumcision staplers.

### BACKGROUND

With the development of medicine and product research and development, an increasing number of minimally invasive medical devices are being used in urology and andrology clinical operations, with the circumcision stapler being one of them. The use of a circumcision stapler has many advantages such as less intraoperative bleeding, shorter operation time, minimizing manual errors, reducing postoperative infection, less postoperative pain and faster functional rehabilitation. However, the existing circumcision stapler, when in use, is relatively complicated to operate; and the remaining length of the frenulum of prepuce is determined based on the doctor's experience during the operation, thereby requiring the doctor to have rich clinical experience, and sometimes medical disputes may be caused if the operation is not carried out precisely enough.

### SUMMARY

The circumcision stapler, when in use, is relatively complicated to operate; doctors are required to have a high-level operating skill and rich experience as they need to manipulate four operating forceps to control and adjust the length of the frenulum and inner layer, and determine the remaining length of the frenulum of prepuce based on their experience; and doctors are required to have rich clinical experience for a situation where the length of the frenulum is too short if the operation is not carried out precisely enough.

In addition, the circumcision stapler, when in use, sutures the prepuce with suturing nails; however, due to the large gap between the suturing nails, there is still a lot of bleeding.

US 2014 263 570 A1 discloses a surgical stapler that includes a jaw assembly at a distal end connected to a handle assembly that is configured to control the stapler and actuate the deployment of staples. US 2008 065 154 A1 discloses a surgical staple that includes a base and at least one tine extending from the base. CN 105 232 100 A discloses a suturing nail which is rapid to fall through a physiological reaction in a human body. WO 2015 010 477 A1 discloses a suturing mechanism of a circumcision stapler comprising a glans mask and a plurality of staples, wherein the glans mask comprises a glans hood body, and a proximal end of the glans hood body is provided with an annular recess provided with a plurality of circumferentially and evenly spaced nail seat positioning projections. WO 2015 010 477 A1 does not disclose the glans hood body having a nail plate comprising an annular plate body with evenly spaced inner nail slots and outer nail slots, wherein the plurality of outer nail slots are arranged on the outer side of the plurality of inner nail slots, and there is a spacing area between two of the adjacent inner nail slots, with the corresponding outer nail slot being able to cover at least the spacing area; and the staples having a further pair of outer pins arranged on an outer side of the floor, the pair of outer pins are arranged on an outer side of the pair of inner pins and comprising a third pin and a fourth pin arranged on the left and right sides of the floor respectively.

It is an object of present invention to provide a suturing mechanism of a circumcision stapler and circumcision stapler enabling less bleeding or even no bleeding during and after the circumcision.

### TECHNICAL SOLUTION

The present invention is defined by the independent claim. Advantageous embodiments are described in the dependent claims, the following description and the drawings.

The present invention provides a suturing mechanism of a circumcision stapler, wherein the suturing structure comprises a glans holder and a plurality of the suturing nails described below, wherein the glans holder comprises a glans cover, a proximal end of the glans cover is provided with an annular recess, the annular recess is provided with a nail plate, and the nail plate comprises an annular plate body; the plate body is provided with a plurality of circumferentially and evenly spaced inner nail slots and outer nail slots, wherein the plurality of outer nail slots are arranged on the outer side of the plurality of inner nail slots, and there is a spacing area between two of the adjacent inner nail slots, with the corresponding outer nail slot being able to cover at least the spacing area; the suturing nail configured to suture a prepuce through the nail plate, that is, the suturing work of the suturing nail provided by the present invention is completed through the adapted glans cover.

In some embodiments, the glans cover defines a distally open glans cavity, and an inner wall of the glans cavity is provided with a protrusion extending from a proximal end to a distal end; the proximal end of the glans cavity is folded outward to form an annular recess with an opening downward, and a center of the protrusion is provided with a limiting slot for fixing a frenulum.

In some embodiments, a plurality of hooks are circumferentially and evenly spaced on an outer wall of the glans cover, and the hooks are provided from a proximal end to a distal end with a plurality of hooking parts; preferably, the glans holder further comprises a plurality of sutures, with one end of the sutures arranged on the hooks and the other end configured for threading through the prepuce; the hooks, coordinated with the sutures, are configured to adjust a length of the prepuce to be cut. The pin structure comprises a pair of inner pins arranged on an inner side of the floor and a pair of outer pins arranged on an outer side of the floor, the pair of outer pins are arranged on an outer side of the pair of inner pins, the pair of inner pins comprise a first pin and a second pin arranged symmetrically on left and right sides of the floor, with the first pin and the second pin both extending axially upward from the floor and being able to bend relatively toward each other, and the pair of outer pins comprise a third pin and a fourth pin arranged on the left and right sides of the floor respectively.

In some embodiments, the third pin and the pin of the pair of outer pins can each independently bend inwardly or outwardly.

In some embodiments, the third pin extends toward the left with respect to the floor and the fourth pin extends axially upward from the floor and can bend toward the right with respect to the floor; preferably, the third pin extend horizontally or nearly horizontally.

In some embodiments, the suturing nail further comprises at least one pair of side wings; wherein the pair of side wings comprise two side wings extending laterally outward from the left and right sides of the floor respectively; preferably, the pair of side wings extend horizontally or extend upwardly in a warping manner.

In some embodiments, a tip of at least one of the first pin, the second pin, the third pin and the fourth pin is beveled on a side away from the floor.

The present invention yet still further provides a circumcision stapler, which comprises the suturing mechanism described above.

### BENEFICIAL EFFECTS OF THE INVENTION

In the present invention, a closed suturing of the whole circle of the cutting part is achieved by adding pins on the suturing nails and adjusting the position of the suturing nails, making it possible for less bleeding or even no bleeding during and after the circumcision. Specifically, a closed-loop suturing within the nail is achieved between the inner pins and the floor, and a coordination between the nails is achieved through the bending of the pair of outer pins or the flat pins. In the present invention, a closed suturing of the whole circle of the cutting part is further achieved by adding side wings and adjusting the position of the suturing nails, making it possible for less bleeding or even no bleeding during and after the circumcision.

### BRIEF DESCRIPTION OF THE DRAWINGS

FIG. 1 is a schematic structural diagram of the glans holder according to the present invention;
FIG. 2 is a schematic structural diagram of the glans holder according to the present invention;
FIG. 3 is a schematic diagram of an operation of the glans holder according to the present invention;
FIG. 4 is a schematic structural diagram of the nail plate of the glans holder according to the present invention;
FIG. 5 is a schematic structural diagram of the suturing nail according to the present invention;
FIGs. 6 and 7 are schematic structural diagrams of the bent suturing nail according to the present invention;
FIG. 8 is a schematic diagram of a suture by the suturing nail according to the present invention;
FIGs. 9 and 10 are schematic structural diagrams of the bent suturing nail according to the present invention;
FIG. 11 is a schematic diagram of a suture by the suturing nail according to the present invention;
FIG. 12 is a schematic structural diagram of the bent suturing nail according to the present invention;
FIG. 13 is a schematic diagram of a suture by the suturing nail according to the present invention;
FIGs. 14 and 15 are schematic structural diagrams of the bent suturing nail according to the present invention;
FIG. 16 is a schematic diagram of a suture by the suturing nail according to the present invention;
FIGs. 17 and 18 are schematic diagrams of the structure of and a suture by another suturing nail according to the present invention;
FIGs. 19 and 20 are schematic diagrams of the structure of and a suture by another suturing nail according to the present invention; and
FIGs. 21 to 24 are schematic diagrams of the structure of and a suture by another suturing nail according to the present invention.

### DETAILED DESCRIPTION

Embodiments of the present invention are described in detail below, and examples of the embodiments are shown in the drawings, where the same or similar reference numbers throughout the article indicate the same or similar elements or elements with same or similar functions. The embodiments described below with reference to the drawings are examples and are intended to explain the present invention and should not be construed as limitations on the present invention.

In describing the present invention, it is to be understood that direction and position relations indicated by terms such as "up", "down", "front", "rear", "left", "right", "vertical", "horizontal", "top", "bottom", "inside", "clockwise" and "counterclockwise" are based on the direction and position relations shown in the drawings, and these terms are used merely to facilitate and simplify the description of the present invention, instead of indicating or implying that the device or element referred to must have a particular direction and be constructed and operated in a particular direction, and therefore, should not be construed as limitations on the present invention.

In the present invention, terms such as "mount", "attach", "connect", "fix" and "hold" are to be understood in a broad sense unless otherwise explicitly specified and limited; for example, a connection may be a fixed connection, a removable connection, or an integral connection; it may be a mechanical connection or an electrical connection; and it may be a direct connection, an indirect connection through an intermediate medium, or a connection of the inner segments of two elements. For those of ordinary skill in the art, the specific meanings of the terms described above in the present invention can be understood according to specific conditions.

In order to make the objectives, technical solutions and advantages of the present invention more apparent, the present invention is further described in detail below with reference to the drawings and embodiments. It should be understood that the specific embodiments described herein are merely illustrative of the present invention and do not limit the present invention.

### Embodiment 1

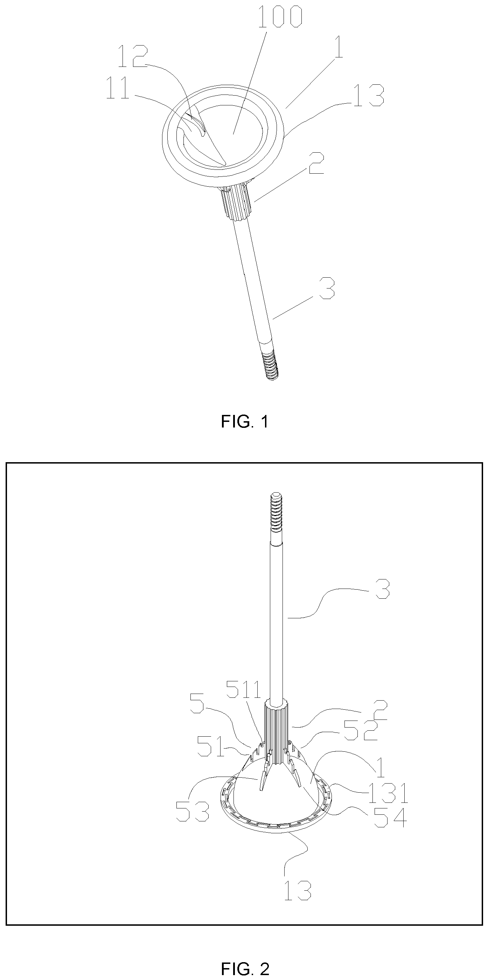

Referring to FIGs. 1-2, the embodiment provides a glans holder for a circumcision stapler, wherein the glans holder comprises a glans cover 1, a connecting rod 2 arranged at the proximal end of the glans cover 1 and a screw rod 3 connected to the connecting rod 2. The glans holder can be driven to move back and forth by coordinating the screw rod and a knob. The glans cover 1 defines a distally open glans cavity 100 configured for accommodating the glans for circumcising the external prepuce by the circumcision stapler, and the inner wall of the glans cavity 100 is provided with a protrusion 11 extending from the proximal end to the distal end. In this way, the glans holder according to the embodiment increases the overall climbing length for the frenulum compared to a glans holder with a smooth inner wall, allowing more frenulum to remain within the holder. In addition, the protrusion increases a corresponding distance for the prepuce as it starts to round the outer ring of the glans holder at the center of the bell, ensuring that the prepuce does not become too short after the circumcision. Meanwhile, to avoid the displacement of the frenulum, a limiting part for fixing the frenulum is arranged on the protrusion. There are various configurations for the limiting part, and in the embodiment, the configuration of the limiting part is as follows: a limiting slot 12 for fixing the frenulum is arranged at the center of the protrusion so that the frenulum can be taken in the limiting slot 12. In this way, it can be ensured that the frenulum will not wander freely during the operation while increasing the climbing distance for the frenulum, further ensuring that the frenulum would not be excised too short. In the embodiment, the protrusion 11 is a protruding strip extending from the proximal end of the glans cavity to the distal end that effectively increases the climbing distance for the frenulum, and the protruding strip is provided with a curved surface to avoid damage to the frenulum.

### Embodiment 2

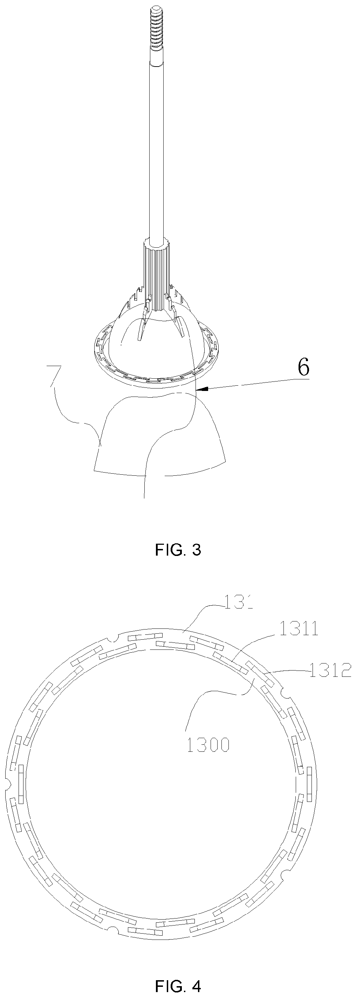

Referring to FIGs. 1-2, the embodiment provides a glans holder for a circumcision stapler, wherein the glans holder comprises a glans cover 1, a connecting rod 2 arranged at the proximal end of the glans cover 1 and a screw rod 3 connected to the connecting rod 2. The glans cover 1 defines a distally open glans cavity 100, and the proximal end of the glans cover 1 is folded downward to form an annular recess 13. A plurality of hooks 5 are arranged circumferentially at the proximal side of the glans holder for sutures to wind thereon. In the embodiment, one end of the suture 6 threads through the prepuce 7 with a needle and is secured by a knot, while the other end is pulled on the hooks 5. In this way, the prepuce can be lifted by threading multiple sutures circumferentially thereon. This procedure ensures that the frenulum is loose by pulling the sutures to properly tight the inner layer so as to ensure that the frenulum is not excised too much, and allows the sutures to completely replace the use of hemostatic forceps, eliminating the need for multiple hemostatic forceps to clamp the prepuce, making the operation simpler. In addition, the large size of the hemostatic forceps is not conducive to the extension of the glans holder into the glans, whereas the multiple sutures according to the embodiment for lifting the prepuce have a small size, which hardly causes any adverse effect on the extension of the glans holder. In the embodiment, a plurality of hooks 51, 52, 53, 54 are circumferentially and evenly spaced on the outer wall of the glans cover, so that the circumference of the prepuce is evenly lifted. To ensure an easy operation, the hooks are provided from the proximal end to the distal end with a plurality of hooking parts 511, and the plurality of hooking parts make it easy for a doctor to hang and lock the sutures to enable the doctor, after adjusting the length of the prepuce, to fix the sutures for the subsequent circumcising operation. Specifically, the glans holder further comprises a plurality of sutures, with one end of the sutures arranged on the hooks and the other end configured for threading through the prepuce. Referring to FIG. 3, the sutures used by a doctor according to the present invention thread through the prepuce, with one end being secured to the prepuce and the other end being pulled to adjust the length of the inner layer and frenulum of the prepuce. The multiple hook structures make it easy for a doctor to hang and lock the sutures, allowing the sutures to be adjusted simultaneously in multiple directions and the length of the prepuce to be adjusted at any time.

### Embodiment 3

Referring to FIG. 2 and FIG. 4, in the embodiment, the bell base is improved on the basis of Embodiment 1. The proximal end of the glans cavity is folded downward to form an annular recess 13, the annular recess 13 is provided with a nail plate 131, and the nail plate 131 comprises an annularly formed plate body. The plate body is provided with a plurality of circumferentially and evenly spaced inner nail slots 1311 and outer nail slots 1312, wherein the plurality of outer nail slots 1312 are arranged on the outer side of the plurality of inner nail slots 1311, and there's a spacing area 1300 between two of adjacent inner nail slots 1311, with the corresponding outer nail slot 1312 being able to cover at least the spacing area 1300. In this way, the outer nail slot just cover the spacing area of any two adjacent inner nail slots corresponding thereto, forming a 360° closed loop and thus realizing a double protection, so that the intraoperative and postoperative bleeding is significantly reduced.

### Embodiment 4

The embodiment provides a suturing nail, wherein the suturing nail comprises a floor 8, and a pin structure and two side wings arranged on the floor 8. The pin structure comprises a pair of inner pins and a pair of outer pins extending axially upward from the floor respectively, wherein the pair of inner pins are positioned on the inner side of the pair of outer pins, the pair of inner pins comprise a first pin 9A and a second pin 9B arranged symmetrically in the left and right direction, and the pair of outer pins comprise a third pin 10A and a fourth pin 10B arranged on the left and right sides of the floor respectively, with the pins of the pair of inner pins and the pair of outer pins each being independently disposed to be able to bend inwardly or outwardly. The two side wings 81, 82 extend laterally outward from the left and right sides of the floor respectively. In this way, the effect of using two rows of suturing nails is obtained without increasing the number of the suturing nails, and bleeding can be well inhibited without increasing the difficulty of operation.

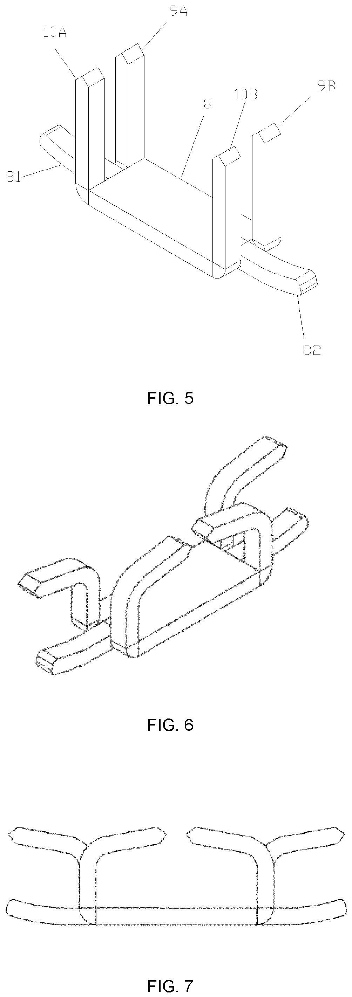

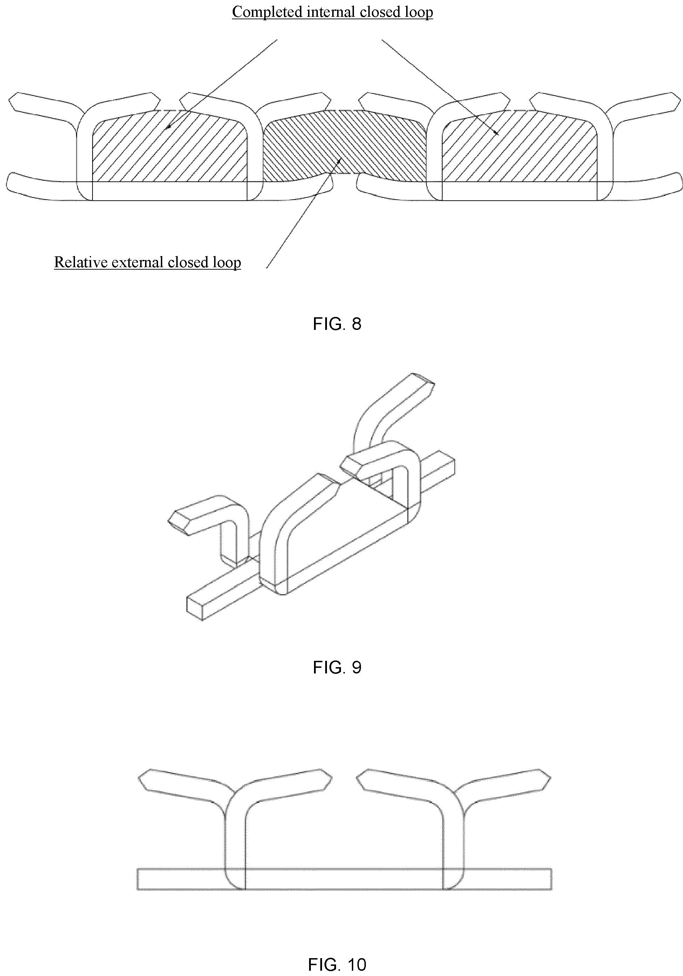

For example, the embodiment provides the following suturing methods:
Structure 1-Staggered warping suturing: as shown in FIGs 6 and 7, the side wings extend upwardly in a warping manner, and the pins of the suturing nail, in the process of suturing, bend along the nail slots of the nail plate of the glans holder, with the two inner pins bending inwardly and the two outer pins bending outwardly on each side. The two inner pins bend inwardly to form, with the floor of the suturing nail, a clamped closed loop, and the prepuce tissue therein will be in a compressed state, preventing the tissue therein from outward capillary hemorrhage, which is the main barrier to prevent capillary hemorrhage, as shown in FIG. 8. The two outer pins bend outwardly on each side to form, with the side wings on the same side, a "semi-closed loop", and the adjacent suturing nails form a "semi-closed loop" in the same principle, the two "semi-closed loops" forming a relatively closed loop. The pins that bend back, together with the side wings that wrap upward, partly clamp the prepuce within them, which is the secondary barrier to prevent capillary hemorrhage.

The suturing nail is provided with four pins, two on the inner side and two on the outer side, and is further provided with two upwardly warped side wings in the axial direction to increase pretightening force.

Specifically, the pair of side wings extend outward from the middle in a front-to-back direction of the left and right sides of the floor respectively.

### Structure 2-Staggered horizontal suturing:

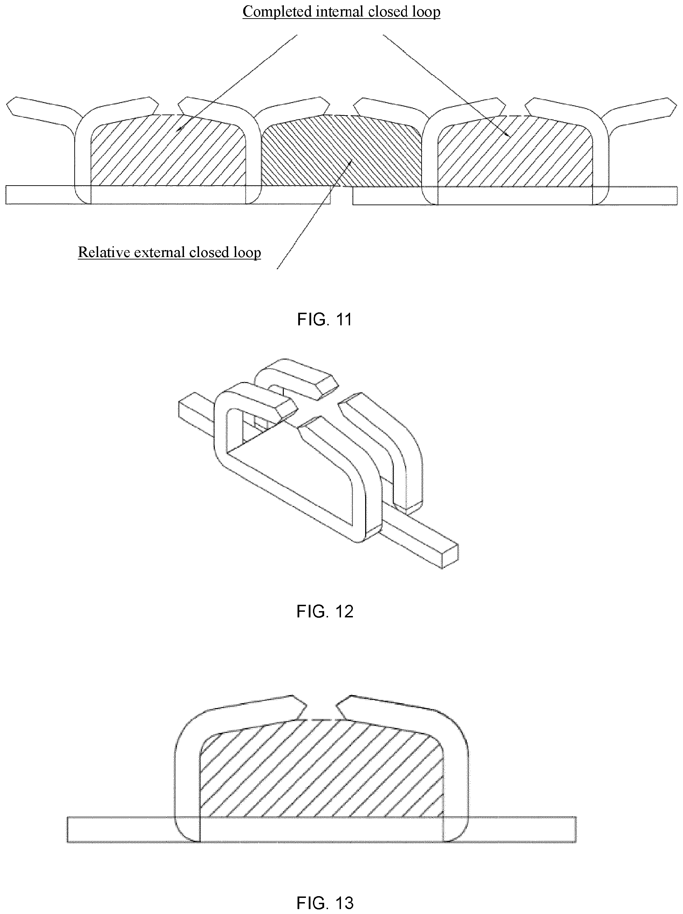

As shown in FIGs. 9 and 10, the pins of the suturing nail, in the process of suturing, bend along the nail slots of the nail plate of the bell base, with the two inner pins bending inwardly and the two outer pins bending outwardly on each side. The two inner pins bend inwardly to form, with the floor of the suturing nail, a clamped closed loop, and the prepuce tissue therein will be in a compressed state, preventing the tissue therein from outward capillary hemorrhage, which is the main barrier to prevent capillary hemorrhage, as shown in FIG. 11. The two outer pins bend outwardly on each side to form, with the side wings on the same side, a "semi-closed loop", and the adjacent suturing nails form a "semi-closed loop" in the same principle, the two "semi-closed loops" forming a relatively closed loop. The pins that bend back, together with the horizontal side wings, partly clamp the prepuce within them, which is the secondary barrier to prevent capillary hemorrhage.

### Structure 3-Side-by-side inward suturing:

As shown in FIGs. 12 and 13, the pins of the suturing nail, in the process of suturing, bend along the nail slots of the nail plate of the bell base, with the four inner and outer pins all bending inwardly. The two inner pins bend inwardly to form, with the floor of the suturing nail, a clamped closed loop, and the prepuce tissue therein will be in a compressed state, preventing the tissue therein from outward capillary hemorrhage, which is the first layer of the barrier to prevent capillary hemorrhage. The two outer pins bend inwardly in the same principle to form a clamped closed loop, which is the second layer of the barrier to prevent capillary hemorrhage.

### Structure 4-Side-by-side outward suturing:

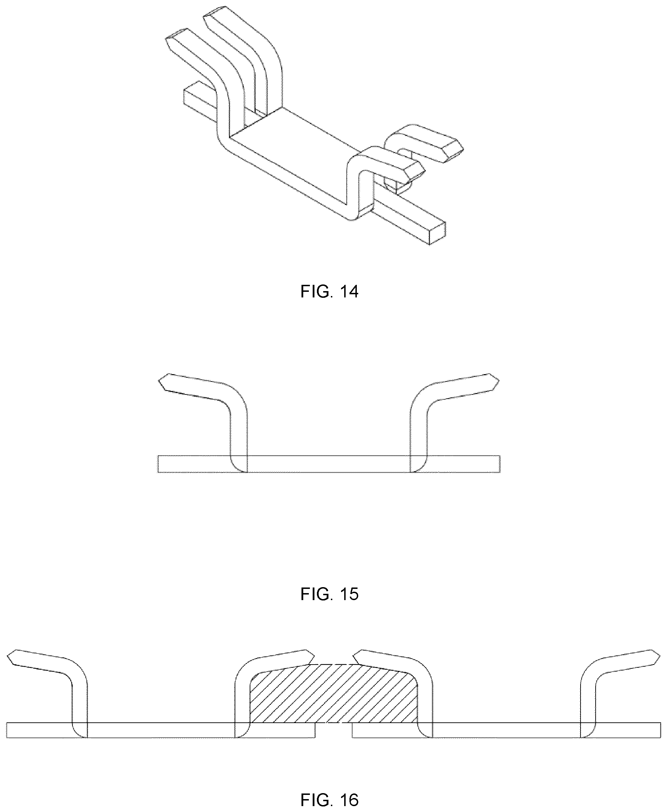

As shown in FIGs. 14 to 15, the pins of the suturing nail, in the process of suturing, bend along the nail slots of the nail plate of the bell base, with the four inner and outer pins all bending outwardly. As shown in FIG. 16, the two inner pins bend inwardly to form, with the side wings of the suturing nail, a "semi-closed loop", and the adjacent suturing nails form a "semi-closed loop" in the same principle, the two "semi-closed loops" forming a relatively closed loop. The pins that bend back, together with the horizontal side wings, partly clamp the prepuce within them, which is the first layer of the barrier to prevent capillary hemorrhage. The two outer pins bend outwardly in the same principle to form a relatively clamped closed loop, which is the second layer of the barrier to prevent capillary hemorrhage.

With the suturing nails provided by the present invention, the effect of using two rows of suturing nails may be obtained without increasing the number of the suturing nails, and bleeding can be well inhibited without increasing the difficulty of operation.

### Embodiment 5

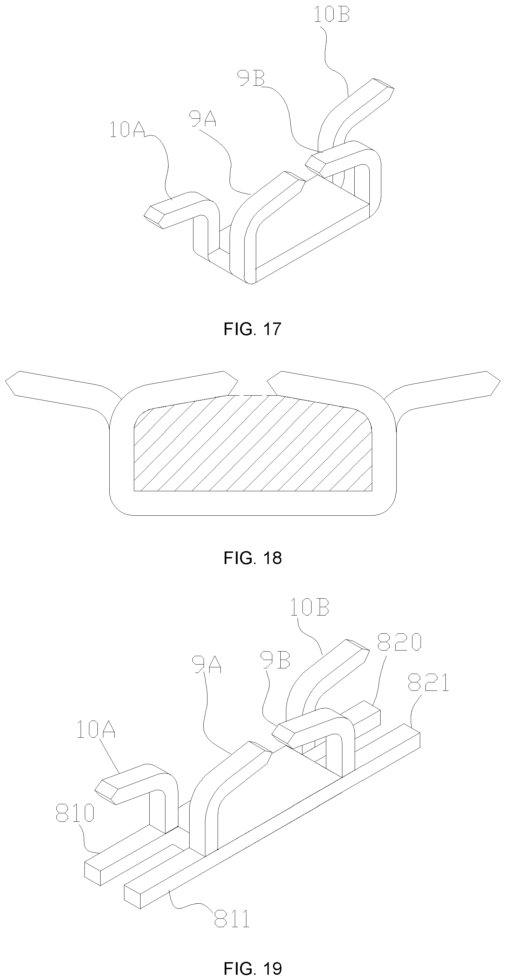

Referring to FIG. 17, the embodiment provides a suturing nail, wherein the suturing nail comprises a floor 8, and a pin structure arranged on the floor 8. The pin structure comprises a pair of inner pins and a pair of outer pins extending axially upward from the floor respectively, wherein the pair of inner pins are positioned on the inner side of the pair of outer pins, the pair of inner pins comprise a first pin 9A and a second pin 9B arranged symmetrically in the left and right direction, and the pair of outer pins comprise a third pin 10A and a fourth pin 10B arranged symmetrically in the left and right direction, with the first pins and the second pins of the pair of inner pins and the pair of outer pins each being independently disposed to be able to bend inwardly or outwardly. In this way, the effect of using two rows of suturing nails is obtained without increasing the number of the suturing nails, and bleeding can be well inhibited without increasing the difficulty of operation.

Specifically, referring to FIG. 18, the first pin 9A and the second pin 9B of the pair of inner pins bend relatively toward each other to form, with the floor, a closed loop. The third pin 10A on the left side and the fourth pin 10B on the right side bend toward the left and right sides respectively to form, with the adjacent suturing nails, external closed loops. By addition of one more row of pins to the conventionally one-row pins, inhibition of bleeding is remarkably improved.

### Embodiment 6

Referring to FIG. 19, the embodiment provides a suturing nail, wherein the suturing nail comprises a floor 8, and a pin structure and two pairs of side wings arranged on the floor 8. The pin structure comprises a pair of inner pins and a pair of outer pins extending axially upward from the floor respectively, wherein the pair of inner pins are positioned on the inner side of the pair of outer pins, the pair of inner pins comprise a first pin 9A and a second pin 9B arranged symmetrically in the left and right direction, and the pair of outer pins comprise a third pin 10A and a fourth pin 10B arranged symmetrically in the left and right direction, with the first pins and the second pins of the pair of inner pins and the pair of outer pins each being independently disposed to be able to bend inwardly or outwardly;
the two pairs of side wings comprise a pair of outer side wings and a pair of inner side wings, wherein the pair of outer side wings are positioned on the outer side of the pair of inner side wings, the pair of outer side wings comprise a side wing 810 and a side wing 820 extending laterally outward from the left and right sides of the floor respectively; and the pair of inner side wings comprise a side wing 811 and a side wing 821 extending laterally outward from the left and right sides of the floor respectively.

Two pairs of side wings further enhance the inhibition of bleeding compared to one pair of side wings.

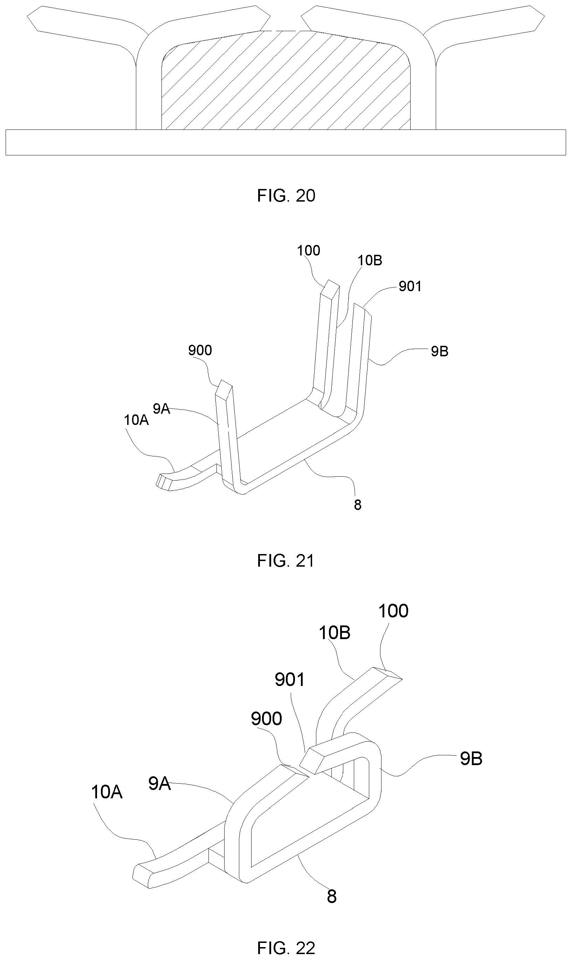

For example, referring to FIG. 20, the pins of the suturing nail, in the process of suturing, bend along the nail slots of the nail plate of the glans holder, with the two inner pins bending inwardly and the two outer pins bending outwardly on each side. The two inner pins bend inwardly to form, with the floor of the suturing nail, a clamped closed loop, and the prepuce tissue therein will be in a compressed state, preventing the tissue therein from outward capillary hemorrhage, which is the main barrier to prevent capillary hemorrhage. The two outer pins bend outwardly on each side to form, with the two side wings on the same side, a "semi-closed loop", and the adjacent suturing nails form a "semi-closed loop" in the same principle, the two "semi-closed loops" forming a relatively closed loop. The pins that bend back, together with the side wings, partly clamp the prepuce within them, which is the secondary barrier to prevent capillary hemorrhage.

In this way, the effect of using two rows of suturing nails may be obtained without increasing the number of the suturing nails, and bleeding can be well inhibited without increasing the difficulty of operation.

### Embodiment 7

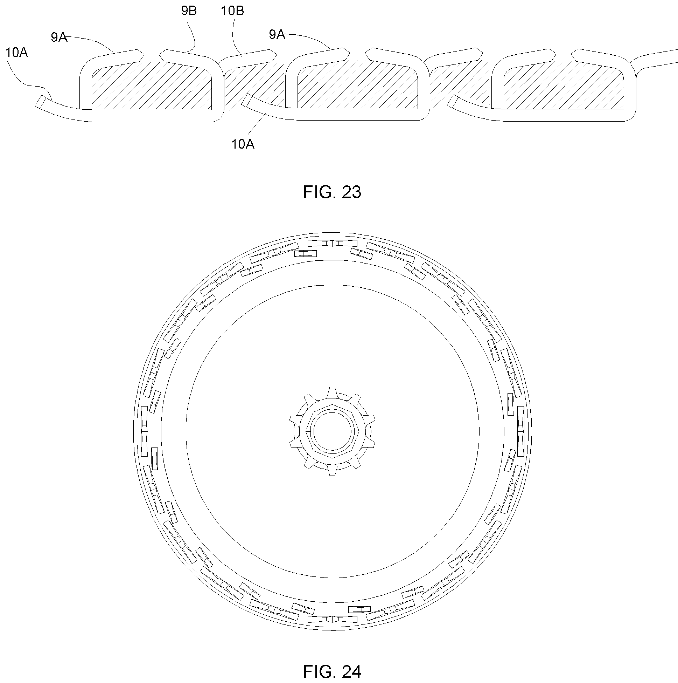

Referring to FIGs. 21 and 22, the embodiment provides a suturing nail for a circumcision stapler, wherein the suturing nail comprises a floor 8 and a pin structure arranged on the floor 8. The pin structure comprises a pair of inner pins arranged on the inner side of the floor and a pair of outer pins arranged on the outer side of the floor, and specifically, the pair of outer pins are arranged on the outer side of the pair of inner pins. The pair of inner pins comprise a first pin 9A and a second pin 9B arranged symmetrically on the left and right sides of the floor, wherein the first pin 9A and the second pin 9B both extend axially upward from the floor 8 and are able to bend relatively toward each other, referring to FIG. 22 for details, and the first pin 9A and the second pin 9B bend introversively toward each other to compress the tissue, completing the suturing within the nail;
the pair of outer pins comprise a third pin 10A and a fourth pin 10B arranged on the left and right sides of the floor respectively, wherein the third pin 10A extends toward the left with respect to the floor and the fourth pin extends axially upward from the floor and is able to bend toward the right with respect to the floor. Referring again to FIGs. 23 and 24, the fourth pin 10B bends extroversively so as to be able to suture with the adjacent third pin 10A of the suturing nail, such that the suture is completed and bleeding is stopped;
referring to FIGs 23 and 24, the part between the two suturing nails is compressed by the pin of the left suturing nail that is bent extroversively and the flat pin of the right suturing nail, forming a whole circle, such that the suture is completed and bleeding is stopped. Specifically, the third pin 10A extends horizontally or nearly horizontally, and in the embodiment, the free end of the third pin 10A warps upward with respect to the floor to better wrap the sutured tissue, thus completing the suturing.

The tips of the first pin 9A and the second pin 9B are beveled on the sides away from the floor to enable an inward suturing of the tissue when the first pin 9A and the second pin 9B bend relatively toward each other. The tip of the fourth pin 10B is beveled on the side facing the floor, and the beveled side can enhance the piercing and at the same time facilitate the formation of the suturing nail.

The number of nails provided by the present invention is 4, and the manufacturing method and the difficulty of implementation are greatly reduced.

### Embodiment 8

The present invention provides a circumcision stapler, wherein the circumcision stapler comprises the glans holder according to Embodiment 1 or Embodiment 2 or the glans holder according to Embodiment 3 or the suturing nail according to Embodiment 4 or Embodiment 5 or Embodiment 6 or Embodiment 7.

It should be noted that the above embodiments are merely representative examples of the present invention. The present invention may also have many variations.

## Claims

1. A suturing mechanism of a circumcision stapler, wherein the suturing mechanism comprises a glans holder and a plurality of suturing nails, wherein the glans holder comprises a glans cover (1), a proximal end of the glans cover (1) is provided with an annular recess (13), the annular recess (13) is provided with a nail plate (131), and the nail plate (131) comprises an annular plate body; the plate body is provided with a plurality of circumferentially and evenly spaced inner nail slots (1311) and outer nail slots (1312), wherein the plurality of outer nail slots (1312) are arranged on the outer side of the plurality of inner nail slots (1311), and there is a spacing area (1300) between two of the adjacent inner nail slots (1311), with the corresponding outer nail slot being able to cover at least the spacing area (1300); wherein the suturing nails each comprise a floor (8) and a pin structure arranged on the floor (8), wherein the pin structure comprises a pair of inner pins arranged on an inner side of the floor (8) and a pair of outer pins arranged on an outer side of the floor (8), the pair of outer pins are arranged on an outer side of the pair of inner pins, the pair of inner pins comprise a first pin (9A) and a second pin (9B) arranged symmetrically on left and right sides of the floor (8), with the first pin (9A) and the second pin (9B) both extending axially upward from the floor (8) and being able to bend relatively toward each other, and the pair of outer pins comprise a third pin (10A) and a fourth pin (10B) arranged on the left and right sides of the floor (8) respectively, and wherein the suturing nails are configured to suture a prepuce (7) through the nail plate (131).

2. The suturing mechanism according to claim 1, wherein, the glans cover (1) defines a distally open glans cavity (100), and an inner wall of the glans cavity (100) is provided with a protrusion (11) extending from a proximal end to a distal end; the proximal end of the glans cavity (100) is folded outward to form the annular recess (13) with an opening downward, and a center of the protrusion (11) is provided with a limiting slot (12) for fixing a frenulum.

3. The suturing mechanism according to claim 2, wherein, a plurality of hooks (5, 51, 52, 53, 54) are circumferentially and evenly spaced on an outer wall of the glans cover (1), and the hooks (5, 51, 52, 53, 54) are provided from a proximal end to a distal end with a plurality of hooking parts (511); preferably, the glans holder further comprises a plurality of sutures (6), with one end of the sutures (6) arranged on the hooks (5, 51, 52, 53, 54) and the other end configured for threading through the prepuce (7); the hooks (5, 51, 52, 53, 54), coordinated with the sutures (6), are configured to adjust a length of the prepuce (7) to be cut.

4. The suturing mechanism according to claim 1, wherein the third pin (10A) and the fourth pin (10B) of the pair of outer pins can each independently bend inwardly or outwardly.

5. The suturing mechanism according to claim 1, wherein the third pin (10A) extends toward the left with respect to the floor (8) and the fourth pin (10B) extends axially upward from the floor (8) and can bend toward the right with respect to the floor (8); preferably, the third pin (10A) extends horizontally or nearly horizontally.

6. The suturing mechanism according to claim 1, wherein, the suturing nail further comprises at least one pair of side wings (81, 82, 810, 811, 820, 821), wherein the pair of side wings (81, 82, 810, 820) comprise two side wings (81, 82, 810, 811, 820, 821) extending laterally outward from the left and right sides of the floor (8) respectively; preferably, the pair of side wings (81, 82, 810, 811, 820, 821) extend horizontally or extend upwardly in a warping manner.

7. The suturing mechanism according to claim 5, wherein a tip of at least one of the first pin (9A), the second pin (9B), the third pin (10A) and the fourth pin (10B) is beveled on a side away from the floor (8).

8. A circumcision stapler, comprising the suturing mechanism according to any one of claims 1 to 7.

## Patentansprüche

1. Ein Nahtmechanismus für einen Zirkumzisionsstapler, wobei der Nahtmechanismus eine Eichelhalterung und eine Mehrzahl von Klammern umfasst, wobei die Eichelhalterung eine Eichelabdeckung (1) umfasst, wobei ein proximales Ende der Eichelabdeckung (1) mit einer ringförmigen Vertiefung (13) versehen ist, wobei die ringförmige Vertiefung (13) mit einer Klammerplatte (131) versehen ist und die Klammerplatte (131) einen ringförmigen Plattenkörper umfasst, wobei der Plattenkörper mit einer Mehrzahl von inneren Klammeraufnahmen (1311) und äußeren Klammeraufnahmen (1312) versehen ist, die in Umfangsrichtung gleichmäßig verteilt sind, wobei die Mehrzahl der äußeren Klammeraufnahmen (1312) auf der Außenseite der Mehrzahl der inneren Klammeraufnahmen (1311) angeordnet sind und sich zwischen zwei benachbarten inneren Klammeraufnahmen (1311) ein Abstand (1300) befindet, wobei die entsprechende äußere Klammeraufnahme mindestens den Abstand (1300) abdecken kann, wobei die Klammern jeweils eine Grundplatte (8) und eine auf der Grundplatte (8) angeordnete Nadelsystemstruktur umfassen, wobei die Nadelsystemstruktur ein Paar von inneren Nadeln umfasst, die auf einer inneren Seite der Grundplatte (8) angeordnet sind, sowie ein Paar von äußeren Nadeln, die auf einer äußeren Seite der Grundplatte (8) angeordnet sind, wobei das Paar der äußeren Nadeln auf der Außenseite des Paares der inneren Nadeln angeordnet ist, wobei das Paar der inneren Nadeln eine erste Nadel (9A) und eine zweite Nadel (9B) umfasst, die symmetrisch auf der linken und rechten Seite der Grundplatte (8) angeordnet sind, wobei die erste Nadel (9A) und die zweite Nadel (9B) beide axial von der Grundplatte (8) nach oben erstreckt sind und sich relativ zueinander biegen können, und wobei das Paar der äußeren Nadeln eine dritte Nadel (10A) und eine vierte Nadel (10B) umfasst, die jeweils auf der linken und rechten Seite der Grundplatte (8) angeordnet sind, und wobei die Klammern dazu ausgelegt sind, eine Vorhaut (7) durch die Klammerplatte (131) zu vernähen.

2. Der Nahtmechanismus nach Anspruch 1, wobei die Eichelabdeckung (1) eine distal offene Eichelhöhle (100) definiert und eine Innenwand der Eichelhöhle (100) mit einem Vorsprung (11) versehen ist, der sich von einem proximalen Ende zu einem distalen Ende erstreckt, wobei das proximale Ende der Eichelhöhle (100) nach außen umgefaltet ist, um die ringförmige Vertiefung (13) mit einer nach unten offenen Öffnung zu bilden, und wobei die Mitte des Vorsprungs (11) mit einer Begrenzungsnut (12) zur Fixierung eines Frenulums versehen ist.

3. Der Nahtmechanismus nach Anspruch 2, wobei eine Mehrzahl von Haken (5, 51, 52, 53, 54) in Umfangsrichtung gleichmäßig auf einer Außenwand der Eichelabdeckung (1) verteilt sind, und wobei die Haken (5, 51, 52, 53, 54) von einem proximalen Ende zu einem distalen Ende mit einer Mehrzahl von Hakenelementen (511) versehen sind, wobei vorzugsweise die Eichelhalterung ferner eine Mehrzahl von Nähten (6) umfasst, wobei ein Ende der Nähte (6) an den Haken (5, 51, 52, 53, 54) angeordnet ist und das andere Ende so konfiguriert ist, dass es durch die Vorhaut (7) geführt werden kann, wobei die Haken (5, 51, 52, 53, 54) in Verbindung mit den Nähten (6) dazu ausgelegt sind, die Länge der zu entfernenden Vorhaut (7) anzupassen.

4. Der Nahtmechanismus nach Anspruch 1, wobei die dritte Nadel (10A) und die vierte Nadel (10B) des Paares der äußeren Nadeln jeweils unabhängig nach innen oder nach außen gebogen werden können.

5. Der Nahtmechanismus nach Anspruch 1, wobei die dritte Nadel (10A) sich relativ zur Grundplatte (8) nach links erstreckt und die vierte Nadel (10B) sich axial von der Grundplatte (8) nach oben erstreckt und sich relativ zur Grundplatte (8) nach rechts biegen kann, wobei vorzugsweise die dritte Nadel (10A) horizontal oder nahezu horizontal verläuft.

6. Der Nahtmechanismus nach Anspruch 1, wobei die Klammer ferner mindestens ein Paar von Seitenflügeln (81, 82, 810, 811, 820, 821) umfasst, wobei das Paar der Seitenflügel (81, 82, 810, 820) zwei Seitenflügel (81, 82, 810, 811, 820, 821) umfasst, die sich jeweils seitlich nach außen von der linken und rechten Seite der Grundplatte (8) erstrecken, wobei vorzugsweise das Paar der Seitenflügel (81, 82, 810, 811, 820, 821) horizontal oder in einer nach oben gebogenen Weise verläuft.

7. Der Nahtmechanismus nach Anspruch 5, wobei eine Spitze von mindestens einer der ersten Nadel (9A), der zweiten Nadel (9B), der dritten Nadel (10A) und der vierten Nadel (10B) auf einer von der Grundplatte (8) abgewandten Seite abgeschrägt ist.

8. Ein Zirkumzisionsstapler, umfassend den Nahtmechanismus nach einem der Ansprüche 1 bis 7.

## Revendications

1. Un mécanisme de suture pour une agrafeuse de circoncision, le mécanisme de suture comprenant un support de gland et une pluralité d'agrafes de suture, le support de gland comprenant un capuchon de gland (1), une extrémité proximale du capuchon de gland (1) étant pourvue d'une rainure annulaire (13), la rainure annulaire (13) étant équipée d'une plaque à agrafes (131), et la plaque à agrafes (131) comprenant un corps de plaque annulaire, le corps de plaque étant muni d'une pluralité de fentes intérieures à agrafes (1311) et de fentes extérieures à agrafes (1312) réparties uniformément en circonférence, la pluralité de fentes extérieures à agrafes (1312) étant disposées à l'extérieur de la pluralité de fentes intérieures à agrafes (1311), et une zone d'espacement (1300) étant présente entre deux fentes intérieures à agrafes (1311) adjacentes, la fente extérieure correspondante pouvant couvrir au moins la zone d'espacement (1300), les agrafes de suture comprenant chacune une base (8) et une structure de broches disposée sur la base (8), la structure de broches comprenant une paire de broches intérieures disposées sur un côté intérieur de la base (8) et une paire de broches extérieures disposées sur un côté extérieur de la base (8), la paire de broches extérieures étant disposée à l'extérieur de la paire de broches intérieures, la paire de broches intérieures comprenant une première broche (9A) et une deuxième broche (9B) disposées symétriquement sur les côtés gauche et droit de la base (8), la première broche (9A) et la deuxième broche (9B) s'étendant toutes deux axialement vers le haut à partir de la base (8) et pouvant se plier l'une vers l'autre, et la paire de broches extérieures comprenant une troisième broche (10A) et une quatrième broche (10B) disposées respectivement sur les côtés gauche et droit de la base (8), les agrafes de suture étant conçues pour suturer un prépuce (7) à travers la plaque à agrafes (131).

2. Le mécanisme de suture selon la revendication 1, dans lequel le capuchon de gland (1) définit une cavité du gland (100) ouverte distalement, et une paroi interne de la cavité du gland (100) est pourvue d'une saillie (11) s'étendant d'une extrémité proximale à une extrémité distale, l'extrémité proximale de la cavité du gland (100) étant repliée vers l'extérieur pour former la rainure annulaire (13) avec une ouverture dirigée vers le bas, et un centre de la saillie (11) étant muni d'une fente de limitation (12) pour fixer un frein du prépuce.

3. Le mécanisme de suture selon la revendication 2, dans lequel une pluralité de crochets (5, 51, 52, 53, 54) sont disposés uniformément en circonférence sur une paroi externe du capuchon de gland (1), et les crochets (5, 51, 52, 53, 54) sont pourvus, de l'extrémité proximale à l'extrémité distale, d'une pluralité de parties d'accrochage (511) ; de préférence, le support de gland comprend en outre une pluralité de fils de suture (6), une extrémité des fils de suture (6) étant disposée sur les crochets (5, 51, 52, 53, 54) et l'autre extrémité étant conçue pour être insérée à travers le prépuce (7), les crochets (5, 51, 52, 53, 54), en coordination avec les fils de suture (6), étant conçus pour ajuster la longueur du prépuce (7) à retirer.

4. Le mécanisme de suture selon la revendication 1, dans lequel la troisième broche (10A) et la quatrième broche (10B) de la paire de broches extérieures peuvent chacune se plier indépendamment vers l'intérieur ou vers l'extérieur.

5. Le mécanisme de suture selon la revendication 1, dans lequel la troisième broche (10A) s'étend vers la gauche par rapport à la base (8) et la quatrième broche (10B) s'étend axialement vers le haut à partir de la base (8) et peut se plier vers la droite par rapport à la base (8) ; de préférence, la troisième broche (10A) s'étend horizontalement ou presque horizontalement.

6. Le mécanisme de suture selon la revendication 1, dans lequel l'agrafe de suture comprend en outre au moins une paire d'ailes latérales (81, 82, 810, 811, 820, 821), la paire d'ailes latérales (81, 82, 810, 820) comprenant deux ailes latérales (81, 82, 810, 811, 820, 821) s'étendant latéralement vers l'extérieur à partir des côtés gauche et droit de la base (8) respectivement ; de préférence, la paire d'ailes latérales (81, 82, 810, 811, 820, 821) s'étend horizontalement ou s'élève vers le haut en formant une courbure.

7. Le mécanisme de suture selon la revendication 5, dans lequel une pointe d'au moins l'une des premières broches (9A), des deuxièmes broches (9B), des troisièmes broches (10A) et des quatrièmes broches (10B) est biseautée sur un côté éloigné de la base (8).

8. Une agrafeuse de circoncision, comprenant le mécanisme de suture selon l'une quelconque des revendications 1 à 7.
